(19) Europäisches Patentamt
European Patent Office
Office européen des brevets

(11) EP 4 529 921 A1

(12) EUROPEAN PATENT APPLICATION

(43) Date of publication:
**02.04.2025 Bulletin 2025/14**

(21) Application number: **23020447.1**

(22) Date of filing: **26.09.2023**

(51) International Patent Classification (IPC):
***A61K 9/16*** *(2006.01)* ***A61K 9/50*** *(2006.01)*
***A61K 31/519*** *(2006.01)*

(52) Cooperative Patent Classification (CPC):
**A61K 9/5036; A61K 9/1647; A61K 31/519**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Pharmathen S.A.**
**15351 Pallini Attikis (GR)**

(72) Inventors:
- **KOUTRIS, Efthymios**
**Pallini Attikis**
**P.C. 15351 (GR)**
- **KARAVAS, Evangelos**
**Pallini Attikis**
**P.C. 15351 (GR)**

(54) **SUSTAINED RELEASE MICROPARTICLES AND PROCESS FOR THE PREPARATION THEREOF**

(57) The present invention relates to sustained release microparticle pharmaceutical formulations comprising a hydrophobic drug and a process for the preparation thereof.



Processed by Luminess, 75001 PARIS (FR)

## Description

### TECHNICAL FIELD OF THE INVENTION

The invention is in the field of layer-by-layer coated microparticles based on biodegradable aliphatic polyesters, methods of manufacturing same, and application thereof in sustained release drug delivery.

### BACKGROUND OF THE INVENTION

Tuning the bioavailability and controlling the release rate of therapeutic agents is an ongoing challenge within pharmaceutical technology, towards more effective drug-carriers with enhanced properties. Polymer-based drug delivery systems (DDSs) intended to treat chronic diseases (e.g., cancer, neurodegenerative disorders) are designed to deliver a large dose of an agent to a targeted site in a controlled manner, reducing the dosage frequency and the side effects. Patients are prescribed multiple injections for long time periods, and therefore, sustained release formulations are much needed.

Polyelectrolyte-coating is a strategy widely used in biomedical applications, and particularly particle-based drug delivery, to achieve a desired functionalization and to reinforce certain properties, as for example effectively controlling drug-release and enhancing bioavailability. Well-defined coated particles (in terms of size and shape) can be easily produced via a simple and inexpensive approach by integrating layer-by-layer assembly techniques, such as the deposition of oppositely charged biodegradable and biocompatible polymers. Natural polysaccharides have been becoming increasingly popular in that sense, because of their biocompatibility, stability, non-toxicity, biodegradability, low cost, and the presence of many hydrophilic groups, such as hydroxyl, carboxyl and amino groups in their structure. LbL deposition has the advantage of utilizing mild conditions (i.e. aqueous solutions), which are more favorable to preserve correct protein folding and activity in contrast to organic solvents typically employed in the fabrication of many formulations.

Besides promoting a sustained release profile, the design of the polymer carrier must additionally take into consideration the accumulation of the matrix material in the body, which represents a major drawback that can pose a significant health risk. In need for smarter and more bioactive materials, research has been focused on the development of numerous biobased polymers that hold great potential in terms of their biocompatibility, biodegradability, processability and versatility. In that sense, aliphatic polyesters have attracted research and industry interest, mostly due to their good performance, tunable properties (e.g., crystallinity, mechanical performance, degradation rates) and biocompatibility. The degradation of the polyester's macromolecular chain in vivo prevents its bioaccumulation in the body, reducing thus the risk of long-term toxicity. Poly(lactic acid) (PLA), poly($\varepsilon$-caprolactone) (PCL), poly(alkylene succinate)s and polyglycerol hyperbranched polyesters have already found various applications in the field of pharmaceutical technology, towards a more effective administration of a broad range of active ingredients. Among them, poly(lactic-co-glycolic acid) (PLGA) has attracted significant attention due to its tunable biodegradability and versatility.

### SUMMARY OF THE INVENTION

An object of the present invention to address the above-mentioned issues, namely synthesizing a new PLGA-based aliphatic polyester with enhanced biodegradation rate and utilizing it in the fabrication of a sustained-release microparticle system, and further prolong the drugadministration by integrating a multilayered-polyelectrolyte coating onto the system. In this context, a model drug, trametinib (GSK1120212), was loaded on poly(lactic-co-glycolic acid)/poly(ethylene adipate) (PLGA/PEAd) microparticles and the ability of layer-by-layer (LbL) coating to control drug release and maintain its activity was investigated.

### BRIEF DESCRIPTION OF THE FIGURES

Figure 1 -1H-NMR (a) and 13C-NMR (b) spectra of the synthesized PLGA/PEAd 90/10 copolyester (lines appear in order as presented in the legend).

Figure 2 - FTIR spectra of newly synthesized PLGA/PEAd copolymer, as well as neat PLGA and PEAd

Figure 3 - Hydrolysis rate (measured as % weight loss) of the neat PLGA and the synthesized PLGA/PEAd copolymers, in the presence of Rhizopus delemar and Pseudomonas cepacia lipases

Figure 4 - FTIR spectra of the pure drug, the unloaded PLGA/PEAd microparticles, and the drug-loaded ones (lines appear in order as presented in the legend).

Figure 5 - DSC thermograms of the LbL-coated microparticles (with and without drug), and of the pure GSK1120212 (lines appear in order as presented in the legend).

Figure 6 - XRD patterns indicating the drug encapsulation in amorphous form (lines appear in order as presented in the legend).

Figure 7 - SEM micrograph of the neat spray-dried particles in microscale.

Figure 8 - SEM micrographs of the LbL-coated microparticles after 4-layer coating.

Figure 9 - In vitro release profiles of GSK1120212 from PLGA and PLGA/PEAd microspheres, with and without LbL-coating.

Figure 10 - Schematic illustration of the effect of encapsulated LbL GSK1120212 (~1/5 dilution) on transfected HeLa cells. x-axis depicts the time in hours the encapsulated inhibitor was allowed to release before being added to the cells for 2 h of incubation, y-axis depicts % percentage of cells with green fluorescent signal with $1^{st}$ bar (C), $2^{nd}$ bar (U) and $3^{rd}$ bar (N), where C - cytoplasmic for cytoplasmic signal, U - ubiquitous for diffuse signal and N - nuclear for the nuclear signal.

Figure 11 - Schematic representation of the effect of encapsulated LbL GSK1120212 from the blood plasma of experimental animals on transfected HeLa cells. The x-axis shows the time elapsed in hours after the time t=0 of the administration of the encapsulated inhibitor via subcutaneous surgery to the experimental animals, while the y-axis shows the percentage % of cells with a green, fluorescent signal in the cytoplasm ($1^{st}$ bar - C), diffuse signal in the cytoplasm and nucleus ($2^{nd}$ bar - U), and in the nucleus ($3^{rd}$ bar - N). The analysis was done with P*<0.05, comparing the N values (nuclear signal) of the concentrations with the control value, but also with each other. Values with a statistically significant difference are not noted.

## DETAILED DESCRIPTION OF THE INVENTION

### Preparation of the PLGA-PEAd copolymer

PEAd was prepared by the two-stage melt polycondensation method (esterification and polycondensation) in a glass batch reactor based on a previously published method. In the first stage (esterification), PEAd oligomers were synthesized as follows; proper amounts of adipic acid and 1,2-ethyleneglycol in a 1/1.1 molar ratio were placed in a round-bottom flask that was equipped with a mechanical stirrer, condenser and nitrogen inlet. The polymerization mixture was degassed and purged with nitrogen several times, inserted into a heated salt bath at 180 °C and kept therein under constant stirring at 250 rpm, while gradually raising the reaction temperature up to 220 °C over a period of 4 hours. After complete distillation of the theoretical amount of water, the nitrogen flow was stopped, 400 ppm of TBT (0.05 g/mL in toluene) based on the reactants were added to the mixture and high vacuum (5.0 Pa) was slowly applied to avoid excessive foaming. The temperature was increased to 230 °C (400 rpm) and the polycondensation was carried out for 2 hours. PLGA/PEAd copolymer of 90/10 weight ratio was prepared via reactive melt-mixing method, namely a melt transesterification-induced intermolecular chain exchange reaction. Commercial PLGA pellets along with the proper amount of PEAd were freeze-dried for 24 h using a Scanvac freeze-drier system (Coolsafe 110-4 Pro, Labogen Scandinavia) to remove moisture. Subsequently, the PLGA/PEAd sample with 10 wt % of PEAd was melt-mixed under constant mechanical stirring and high vacuum at 180 °C for 90 min in the presence of 0.05% w/v titanium(IV) butoxide (TBT) catalyst.

### Characterization of PLGA/PEAd copolymer

The synthesis of the new co-polyester was verified via 1H NMR and Fourier-transform infrared (FTIR) spectroscopy. The NMR spectra of the prepared copolymers were collected using an Agilent spectrometer (CA, USA) operating at 500 MHz for protons at room temperature.

Deuterated choloroform ($CDCl_3$) was used as a solvent to prepare solutions of 5% w/v. The number of scans was 32 and 512 for 1H-NMR 13C-NMR, while the sweep width was 6 kHz in all cases. FTIR spectra were recorded using a PerkinElmer FTIR spectrometer (Spectrum 1, Waltman, MA, USA) on KBr containing tablets of the samples. The spectra were obtained over the range of 4000 to 400 $cm^{-1}$, at a resolution of 4 $cm^{-1}$, using 16 co-added scans. All spectra presented are baseline-corrected, normalized, and converted to absorbance mode.

Size exclusion chromatography was applied to determine the molecular weight of the produced copolymer. Measurements were performed on a Waters 600 pump machine, Waters hr1, hr2, hr4, hr4, hr5 columns, Shimadzu rid 10-a detector. Calibration has been done with 9 PS standards with molecular weights of 1,000-300,000. The concentration of the solutions prepared was 20mg/1000μL, the volume of each injection was 150μL, with a flow time of 50 min.

To evaluate the copolymer's enzymatic hydrolysis, films from the materials were prepared (~2mm thickness) using an OttoWeber Type PW 30 hydraulic press (Paul-Otto Weber GmbH, Remshalden, Germany) and placed in petri dishes where 5 mL of phosphate buffer solution (0.2 M, pH 7.4) was added, containing 0.09 mg/mL of Rhizopus delemar and 0.01 mg/mL of Pseudomonas cepacia lipase. The petri dishes were kept at 37.0±1.0 °C in an oven for26 days, while the media were replaced every 24 h. After predetermined time intervals, the films were removed, washed thoroughly with distilled water, and dried at 37 °C in a vacuum oven, until constant weight. The degree of enzymatic hydrolysis was

estimated from the weight loss, as compared to the initial weight of the samples. All experiments were conducted in triplicate.

Scanning electron microscopy (SEM, JEOL JMS-840, Oxford Instruments, Tubney Woods Abingdon, Oxfordshire, UK) was used to evaluate the morphology of the prepared films prior and after enzymatic hydrolysis. Carbon coating was used to cover the obtained films (improvement in the conductivity of the electron beam), while the accelerating voltage, the probe current, and the counting time were set at 20 kV, 45 nA, and 60 s, respectively.

Fabrication of the microparticles via spray drying and LbL-coating

Drug-loaded microparticles were prepared via a spray-drying method. In specific, an appropriate amount of PLGA/PEAd 90/10 was completely dissolved in dichloromethane (DCM) under magnetic stirring, leading to a 2% w/v polymer solution. The GSK1120212 drug was then added in 1:10 drug/polymer ratio. The operating conditions of the spray-drier during the procedure were set to: Aspirator 100%, Gas input 5-6 bar (O2 + N2), and Q flow 30 %. The inlet temperature was 60°C, while the outlet temperature was stabilized at 52 ± 2 °C, while the time needed for the completion of the solution spraying was 5.3 ± 1.2 min. After the entire solution was evaporated, the device was turned off and the microparticles were collected from the collection container in the form of white powder.

The layer-by-layer (LbL) coating was then occurred by performing several successive immersions in chitosan (CHI) and alginate (ALG) aqueous solutions of 0.5 % w/v concentration and pH values 3.27 and 7.45, respectively. After each CHI/ALG layer, the microparticles were collected using centrifugation (10 min at 400 rpm). The produced coated microparticles, after four-layer coating, were then dried overnight at RT and stored at 4°C until further use.

Characterization of drug-loaded microparticles

The physical state of the copolymer and the drug in the prepared microparticles was determined via powder X-ray diffractometry (pXRD) and differential scanning calorimetry (DSC). pXRD patterns of copolymers were recorded using an XRD-diffractometer (Rigaku-Miniiflex II) with a CuKα radiation for crystalline phase identification (λ=0.15405 nm for CuKα). All samples were scanned from 5 to 50° with a scanning rate of 1°/min. DSC studies were performed using a Perkin-Elmer, Pyris Diamond DSC. Briefly, accurately weighted samples (5.0 ± 0.1 mg) were hermetically sealed in aluminum pans and placed in the DSC sample holder. Then the samples were heated from 25°C to 250 °C with a heating rate of 20 °C/min and the various thermal events were recorded using the Pyirs Diamond software. The melting points (Tmelt) were determined as the peak temperature, glass-transition temperature (Tg) was determined as the inflection point temperature, while enthalpy of fusion (ΔHf) was determined as the integrated area of the heat flow curve in all cases. The standard deviations of temperatures and enthalpies determined in this work were not higher than 1.0 °C and 3.0 J/g, respectively. Nitrogen flow (50 mL/min) was applied in order to provide a constant thermal blanket within the DSC cell. The instrument was calibrated for temperature using high purity benzophenone, indium, and tin, while the enthalpic response was calibrated using indium.

The formation of molecular interactions between GSK1120212 and the copolymer was evaluated via FTIR spectroscopy. The FTIR spectra of the samples were received with an FTIR spectrometer (model FTIR-2000, Perkin Elmer, Dresden, Germany) using KBr discs (thickness of 500 μm). The spectra were collected in the range from 4000 to 400 cm-1 at a resolution of 2 cm-1 (total of 64 coadded scans) were baseline corrected and converted into absorbance mode.

The distribution, size and morphology of the particles were studied with a scanning electron microscope (SEM) type FESEM JSM 7610 F PLUS (Field emission scanning electron microscope) with integrated X-ray Energy Dispersive Spectrometer (EDS). and OXFORD AZTEC 1 analytical system. The samples were covered with a thin layer of carbon to achieve good conduction of the electron beam.

The drug loading and EE was measured by dissolving 1.0 mg of NPs in dichloromethane:methanol (1:1 v/v) and determining drug's assay via HPLC (method details are given below). Drug loading, yield, and entrapment efficiency were calculated by using the following equations:

$$Drug\ loading\ (\%) = \frac{weight\ of\ PTX\ in\ NPs}{weight\ of\ NPs} \times 100 \qquad (1)$$

$$Yield\ (\%) = \frac{weight\ of\ NPs}{initial\ weight\ of\ raw\ materials} \times 100 \qquad (2)$$

The in vitro release studies were performed in a DISTEK Dissolution Apparatus I (Dissolution system 2100C, Distek, North Brunswick, NJ, USA) equipped with an autosampler (Evolution 4300, Distek, North Brunswick, NJ, USA).

Appropriate dialysis tubing cellulose membranes were used in all cases. All tests were executed at 37 ± 1 °C with 50 rpm. The dissolution medium was 500 mL of simulated body fluid (SBF) at pH = 7.4. Two milliliters of aqueous solution were withdrawn from the release media at predefined time intervals (i.e., 15, 30, 45 min and 1, 2, 4, 6, 8, 12, 18, 24, 36, 48, 72, 96, 120, 144 and 168 h) and the API was quantified via the HPLC method described below.

Drug content was determined using a Shimadzu HPLC prominence system (Kyoto, Japan), consisting of a degasser (DGU-20A5, Kyoto, Japan), a liquid chromatograph (LC-20 AD, Kyoto, Japan), an autosampler (SIL-20AC, Kyoto, Japan), a UV/Vis detector (SPD-20A, Kyoto, Japan), and a column oven (CTO-20AC, Kyoto, Japan). An Athena-C18, 5 μm, 250 mm × 4.6 mm analytical column was used, while the flow rate was set at 1 mL/min and the column temperature was maintained at 25 °C. A diode array detector was used at 227 nm and the quantification of the API was based on a calibration curve prepared at 20, 10, 5, 2.5, 1 and 0.5 μg/mL API to mobile phase (water/ACN 30/70 v/v).

Impact of the formulation on HeLa cells and BALB/c wild-type mice

The evaluation of the effect of the encapsulated MAPK inhibitor GSK1120212 was first done in HeLa cells through the subcellular localization of GFP-ERF to determine their activity and release rate. The release rate was studied by suspending the compounds in complete cell culture medium at a concentration of 20 mg/ml and a temperature of 37°C. At time intervals of 1, 2, 4, 8, 24, 48 and 72 hours, an amount of the supernatant was removed to identify the active effect of the released drug.

The impact of the drug-loaded microparticles were also studied on wild type mice. For that, BALB/c male mice were used which, according to literature, were administered 5 mg/kg animal weight of free GSK1120212, with an average mouse weight of ~30g. The mouse model was administered 2 mg LbL GSK1120212 via subcutaneous neck surgery. Blood sampling subsequently followed at successive time intervals: 3rd - 24th - 48th - 72nd hour, and plasma isolation. From the plasma for each time point, 10 ul in 1 ml of complete medium were added to each well, with transfected HeLa cells. Finally, cytoplasmic (C), ubiquitous (U) and nuclear (N) cells were measured for their green fluorescence signal.

EXAMPLES

Ethylene glycol (99%), adipic acid (ACS reagent, ≥99.0%) and titanium tetrabutylene (TBT) (97%) were purchased from Sigma-Aldrich (Saint Louis, MO, USA) and used for the synthesis of PEAd. PLGA (with 65/35 w/w lactide to glycolide ratio) was kindly donated by Corbion N.V. (Gorinchem, The Netherlands). MEK1/2 inhibitor Trametinib (GSK1120212) was purchased by Selleck Chemicals (http://www.selleckchem.com). Medium molecular weight Chitosan (CHI) with Mw = 180,000 g/mol (N 85% hydrolyzed), degree of deacetylation 88 - 95%, dynamic viscosity of 1% solution in 1% aqueous acetic acid at 20 °C of 89 mPa·s and particle size ≤ 0.5 mm was purchased from Kraeber & CO GMB. Sodium Alginate (ALG) was purchased from Sigma Aldrich. All other reagents used were of either analytical or pharmaceutical grade and used as received.

EXAMPLE 1

Synthesis of the PLGA/PEAd copolymer 90/10

The successful synthesis of the new co-polyesters was verified via 1H NMR and 13C-NMR (Figure 1). The 1H NMR spectrum of the neat polyesters exhibited all expected characteristic proton resonance signals: 1.66 (CH2), 2.36 (CH2C(O)), 3.83 ppm (CH2OH end-chain groups) and 4.27 ppm (OCH2) for PEAd; 1.55-1.58 ppm (CH3 PLA), 4.82 ppm (CH2 PGA) and 5.17-5.22 ppm (CH PLA) for PLGA. Similarly, the 13C-NMR spectra showed several characteristic peaks at: 173.0 ppm (C=O), 62.1 (OCH2), 33.6 (CH2C(O)) and 24.2 (CH2) for PEAd; 169.2 ppm (C=O PLA), 166.3 ppm (C=O PGA), 68.9 ppm (OCH PLA), 60.8 ppm (OCH2 PGA) and 16.6 (CH3 PLA) for PLGA.

The NMR spectra of neat PEAd polyester presented all the expected characteristic resonance signals: 1.66 (CH2), 2.36 (CH2C(O)), 3.83 ppm (CH2OH end-chain groups) and 4.27 ppm (OCH2) for the proton spectra; 173.0 ppm (C=O), 62.1 (OCH2), 33.6 (CH2C(O)) and 24.2 (CH2) for the carbon spectra. Beside the PEAd signals, the copolymers exhibited the resonance signals corresponding to PLGA (1H: 1.55-1.58 ppm (CH3 PLA), 4.82 ppm (CH2 PGA) and 5.17-5.22 ppm (CH PLA); 13C 166.3 ppm (C=O PGA), 68.9 ppm (OCH PLA), 60.8 ppm (OCH2 PGA) and 16.6 (CH3 PLA)) respectively. In the copolymer spectra, the peak corresponding to the PEAd -CH2OH end-chain groups is not observable indicating that the PEAd chains have successfully reacted according to Scheme 1. Due to the significantly higher molecular weight of the commercial polymers, a corresponding diminution/disappearance of the -CH(CH3)COOH or -CH2(COOH) peaks cannot be observed. For the same reason, new signals corresponding to an ethylene diol segment that is bonded to PEAd on one hand and PLGA on the other are not observable in the spectra of the copolymers.

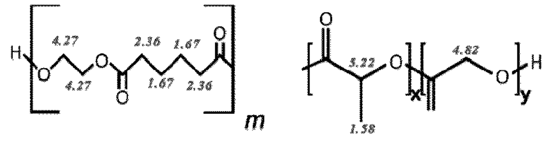

(a)

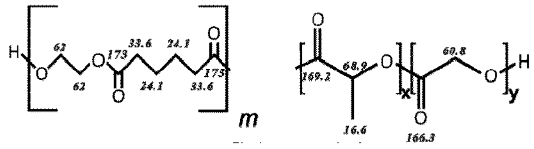

(b)

Scheme 1. NMR signal peaks for the (a) protons and (b) carbons of the PLGA/PEAd copolymer.

Looking at the FTIR spectra (figure 2) of the PLGA-PAEd copolymer in the carbonyl region (-1750 $cm^{-1}$) a double peak is recorded. One of which (i.e., at the lowest wavenumber) corresponds to the carbonyl groups from the PEAd adipic acid, while the second peak corresponds to the C=O groups of PLGA. The existence of these two peaks therefore confirms the presence of both polyesters (i.e., PLGA and PEAd) in the newly synthesized copolymer. Similarly, in the region of methyl and methylene groups (-2900 $cm^{-1}$), three distinct FTIR peaks were observed. In the neat PLGA all three recorded peaks were present, while in the pure PEAd only two of them are recorded, at lower intensities. The fact that the intensities are higher in the final material was expected, since more methylene groups are added in the final copolymer. Finally, the small absorptions seen in the 3600-3300 $cm^{-1}$ region could be attributed to the -OH bonds. These are either due to the unreacted ethylene glycol molecules of PEAd, or due to the hydrogen at the terminal groups of the polymers. These absorptions are higher in the finally synthesized polyesters, which could indicate side reactions between the components during the melt polymerization process and the formation of hydrogen bonds.

The molecular weight values of the produced copolymer were estimated using size exclusion chromatography at different time intervals of the polymerization process (Table 1), leading to a final copolyester of Mw 17600 g/mol with a narrow distribution, suitable for drug delivery applications.

Table 1. Molecular weight values as estimated by SEC.

| Polymerization time (min) | Mn (g/mol) | Mw (g/mol) | PDI |
|---|---|---|---|
| 15 | 12900 | 23600 | 1.8 |
| 30 | 13000 | 22700 | 1.7 |
| 45 | 13500 | 23300 | 1.7 |
| 60 | 8700 | 18100 | 2.1 |
| 90 | 8400 | 17600 | 2.1 |

The main mechanism of drug release from such polyester-based drug delivery systems is polymer's erosion via cleavage (hydrolysis) of the ester groups. Hence, when synthesizing new polyester materials intended for drug matrix/carriers, it is crucial to evaluate their degree of hydrolysis. Therefore, the hydrolysis rate of the neat polyesters and newly synthesized co-polyesters was investigated at 37 °C and pH 7.4, in the presence and absence of enzymes. Specifically, in the present study a blend of Rhizopus delemar and Pseudomonas cepacia lipases was used to investigate the enzymatic hydrolysis of polyester, since these two lipases can be triggered by adsorption on hydrophobic surfaces, and hence can cleave ester bonds in the solid state.

Figure 3 show the % weight loss of the neat PLGA against the PLGA/PEAd copolymer in the presence of lipases. Results indicate that neat PLGA loses only a ~40 % amount of the mass during the study, whereas in the case of the PLGA/PEAd copolymer, the PEAd segment seems to play an important role on the degradation mechanism, showing an initial relatively slow weight loss (the first days), followed by an exponential decrease in weight for the next two weeks, resulting eventually in a significant 67.13% loss.

These results concerning the degradation of the polymer matrix were further evaluated with a morphological study of the sample surface, using scanning electron microscopy. Specifically, based on the obtained micrographs, neat PLGA demonstrated a limited alteration of the film morphology over the course of the 26 days, while the PLGA/PEAd copolymer showed extensive degradation and surface corrosion.

No significant change has been reported at the thermal behavior of the microspheres, too (Figure 5). The recorded Tg value before and after the drug-encapsulation remains unaffected by the encapsulation, which is in accordance with the results from XRD (Figure 6), which reveal the encapsulation of the drug in the amorphous phase, due to the fine molecular dispersion of the drug inside the matrix, as a result of the preparation procedure.

The size of the neat drug-loaded PLGA/PEAd particles were in the order of 1-10 μm, as revealed through SEM studies, while all the formulations had a spherical shape with smooth surfaces (Figure 7). Interesting was the alteration of

the morphology when coating them with the polyelectrolyte system (CHI+ALG). As can be seen, the size of the beads increases slightly progressively as the layers are added, and in addition, it appears that the beads are beginning to aggregate (Figure 8).

EXAMPLE 2

Characterization of the microparticles

The chemical structure of the produced LbL-coated microparticles was studied by FTIR spectroscopy (Figure 4). It can be seen that all the characteristic peaks of the microspheres correspond to the PLGA/PEAd copolymer are present, but it is difficult to distinguish the peaks of GSK1120212 drug, because of the small amount used during the fabrication, but also because the main characteristic bands of the drug overlap with that of the polymer matrix.

EXAMPLE 3

In vitro drug dissolution studies

The present studied system was designed for the prolonged administration of GSK1120212 drug. Table 2 summarizes the results obtained after HPLC analysis for the encapsulation of the drug in the PLGA/PEAd 90/10 microspheres, as well as commercial PLGA (for comparative purposes), with layered coating (LbL-coated) and without coating, in each case. In addition, the in vitro release rate profiles of all the above formulations are listed (Figure 9).

Table 2. % Yield and % drug loading values of the final LbL-coated system (LbL-coated PLGA/PEAd-GSK1120212) compared to its commercial PLGA analogue.

| Microparticles sample | % Yield | % Drug Loading |
|---|---|---|
| uncoated PLGA/GSK1120212 | 72.51 ± 1.42 | 16.32 ± 2.48 |
| LbL-coated PLGA/GSK1120212 | 67.19 ± 1.43 | 15.03 ± 2.87 |
| uncoated PLGA/PEAd-GSK1120212 | 58.2 ± 1.31 | 11.48 ±2.62 |
| LbL-coated PLGA/PEAd-GSK1120212 | 37.40 ± 1.08 | 10.79 ± 3.04 |

The release profiles of all formulations recorded a significant enhancement of the dissolution rate of hydrophobic GSK1120212. The layer-by-layer coating played a key role in terms of the release rate, especially during the first 6-8 hours, as it appears that the CHI/ALG polyelectrolyte complex that constitutes the outer layers of the microsphere successfully controls the "burst effect" (i.e., the sudden release of the active agent upon its first contact with the dissolution medium). The LbL-coated systems further prolong the release of the active substances compared to their uncoated analogues which, although ultimately show higher rates, do not have the desired profile for a sustained administration.

EXAMPLE 4

Cell and animal studies

Figure 10 shows the results of experiments studying encapsulated LbL GSK1120212 (~1/5 dilution) in transfected HeLa cells. The x-axis shows the length of time in hours that the encapsulated LbL inhibitor GSK1120212 was allowed to release before it was added to the cells for 2 h of incubation, while the y-axis shows the percentage % of cells with a green fluorescent signal in the cytoplasm ($1^{st}$ bar - C), diffuse signal in the cytoplasm and nucleus ($2^{nd}$ bar - U), and in the nucleus ($3^{rd}$ bar - N). The analysis was done with P*<0.05, comparing the N values (nuclear signal) of the concentrations with the control value, but also with each other. The statistically significant difference is noted, in relation to the control value at the 24th, 48th and 72nd hours, and between the 1st hour value and the 72nd hour.

By observing the N values of each release snapshot of LbL GSK1120212 (~1/5 dilution) with the N values of each concentration of free GSK1120212 (1-100 nM), it appears that the substance from the LbL capsule is gradually released and at an increasing rate. Looking at the approximate values, the 1 hour value appears to simulate that of 1 nM free substance, the 6 hour value simulates the 4 nM value, the 24 hour value simulates the 5 nM value, the 48 hour value simulates the 8 nM value , while that of the 72nd hour seems to resemble that of 15 nM. Indicatively, it is possible that the release of the substance follows a gradually increasing rate, while 30 nM may have been released from the LbL encapsulation as the last snapshot, as the effect of 15 nM of free substance has no statistically significant difference with the other higher concentrations.

Figure 11 shows the results of experiments studying encapsulated LbL GSK1120212 in wild type mice. The x-axis shows the time elapsed in hours after the time t=0 of the administration of the encapsulated inhibitor via subcutaneous surgery to the experimental animals, while the y-axis shows the percentage % of cells with a green, fluorescent signal in the cytoplasm ($1^{st}$ bar - C), diffuse signal in the cytoplasm and nucleus ($2^{nd}$ bar - U), and in the nucleus ($3^{rd}$ bar - N). The analysis was done with $P^*<0.05$, comparing the N values (nuclear signal) of the concentrations with the control value, but also with each other. Values with a statistically significant difference are not noted.

The data suggest that the release of the drug is probably very limited and a larger amount is required. Note that in all the tests, the effect of the pure blood plasma from the experimental animals on the cells (ctl plasma value) was also examined, in case cellular factors interact with the GSK1120212 inhibitor, causing it to lose its activity. From the results of the experiments, this possibility does not seem to be confirmed.

Nevertheless, the activity of LbL-coated (LbL) GSK1120212-loaded microparticles was clearly superior to its uncoated analogs. In conclusion, the use of GSK1120212 in the context of ERF-associated prostate cancer is promising, as it is a highly specific, reversible inhibitor and extremely low concentrations are required to achieve significant effects.

## Claims

1. Drug delivery formulation comprising a drug and a polymer wherein the polymer is poly(lactic-co-glycolic acid)/-poly(ethylene adipate) (PLGA/PEAd) and the formulation is in the form of coated microparticles providing sustained release.

2. Drug delivery formulation according to claim 1 wherein the poly(lactic-co-glycolic acid)/poly(ethylene adipate) (PLGA/PEAd) polymer has a ration of PLGA to PEAd of 90 to 10.

3. Drug delivery formulation according to any of the preceding claims wherein the microparticles are coated with one or more layers.

4. Drug delivery formulation according to claim 3 wherein the one or more layers are made of chitosan and alginate aqueous solutions.

5. Drug delivery formulation according to claim 4 wherein the one or more layers are made of chitosan and alginate aqueous solutions having a 0.5 % w/v concentration and pH values from 3.00 to 7.50.

6. Drug delivery formulation according to any of the preceding claims wherein the drug is a hydrophobic pharmaceutically active ingredient.

7. A method for encapsulating a drug comprising the steps:

    a. poly(lactic-co-glycolic acid)/poly(ethylene adipate) (PLGA/PEAd) polymer having a ration of PLGA to PEAd of 90 to 10 is completely dissolved in dichloromethane;
    b. drug is then added in 1:10 drug/polymer ratio;
    c. the solution is spray-dried and microparticles are collected; and
    d. the microparticles are immersed one or more times in chitosan and alginate aqueous solutions

    thereby encapsulating said drug into the coated microparticles.

8. A method for encapsulating a drug according to claim 7 wherein the drug is a hydrophobic pharmaceutically active ingredient.

9. A method for encapsulating a drug according to any of claims 7 or 8 wherein the coating made of chitosan and alginate is an aqueous solutions having a 0.5 % w/v concentration and pH values from 3.00 to 7.50.

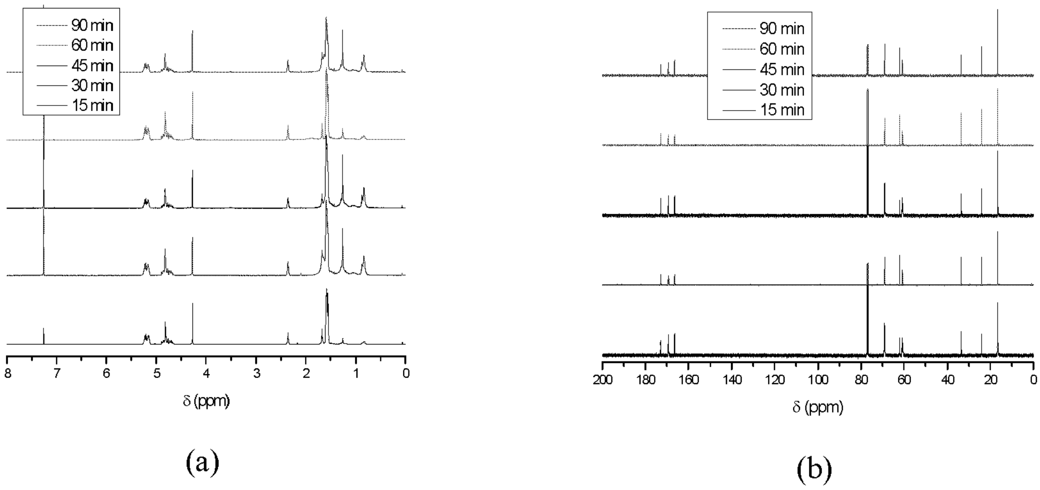


Figure 1

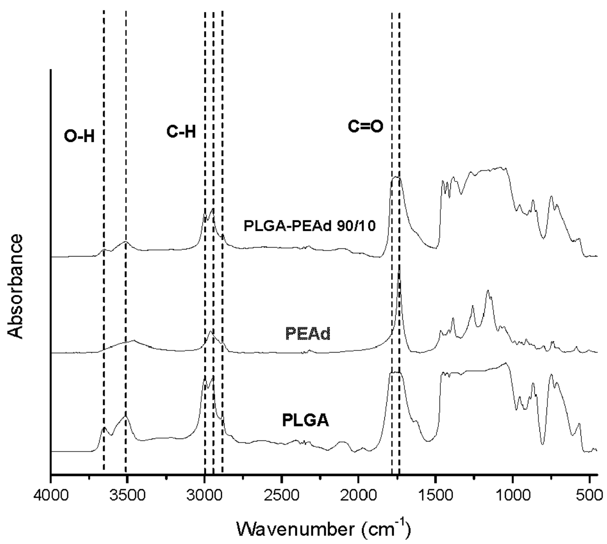


Figure 2

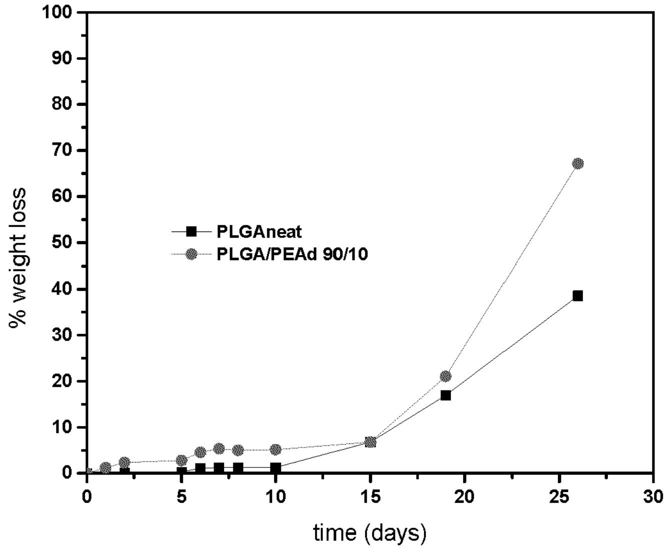


Figure 3

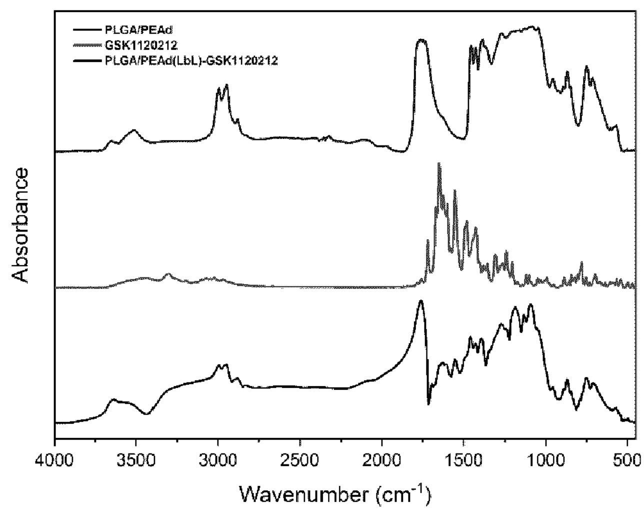


Figure 4

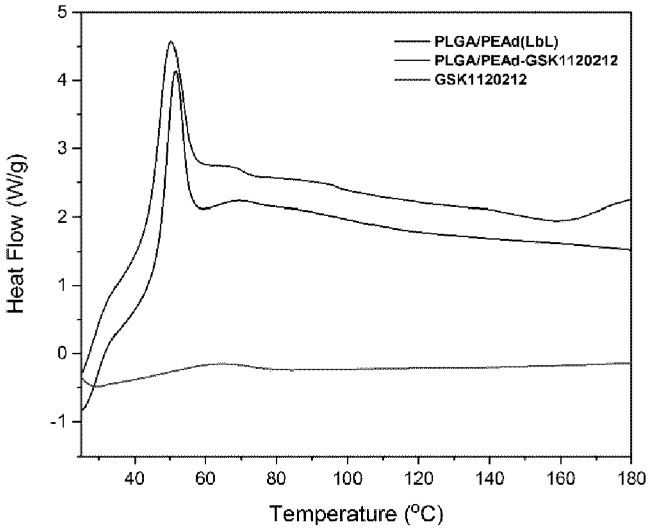


Figure 5

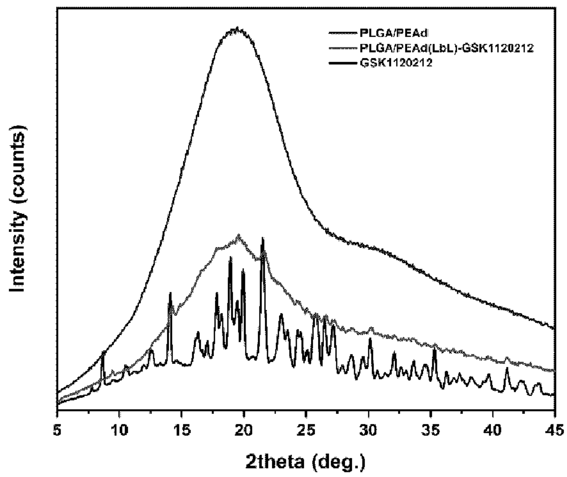


Figure 6

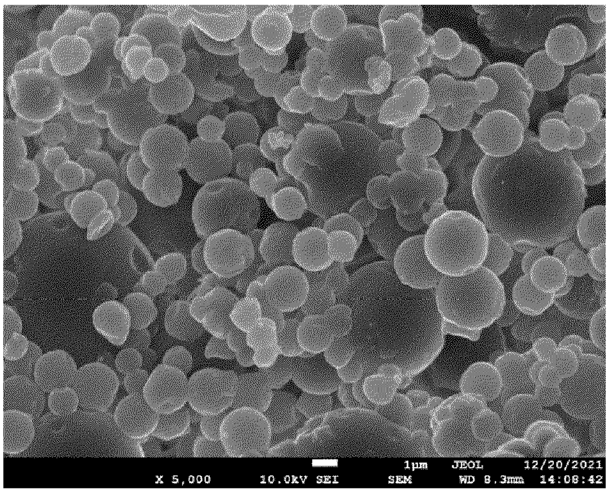


Figure 7

Layer 1 Layer 2 Layer 3 Layer 4

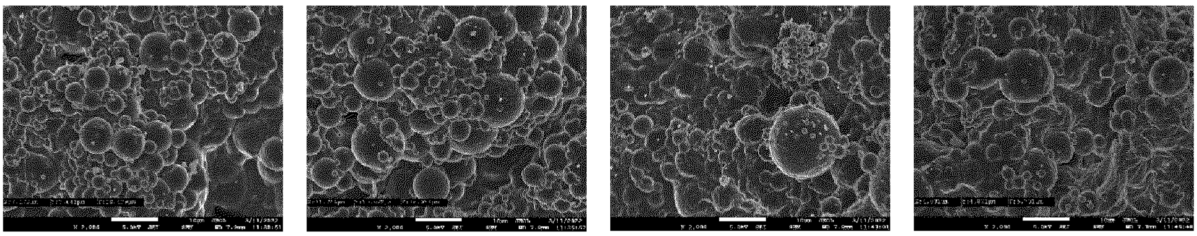

Figure 8

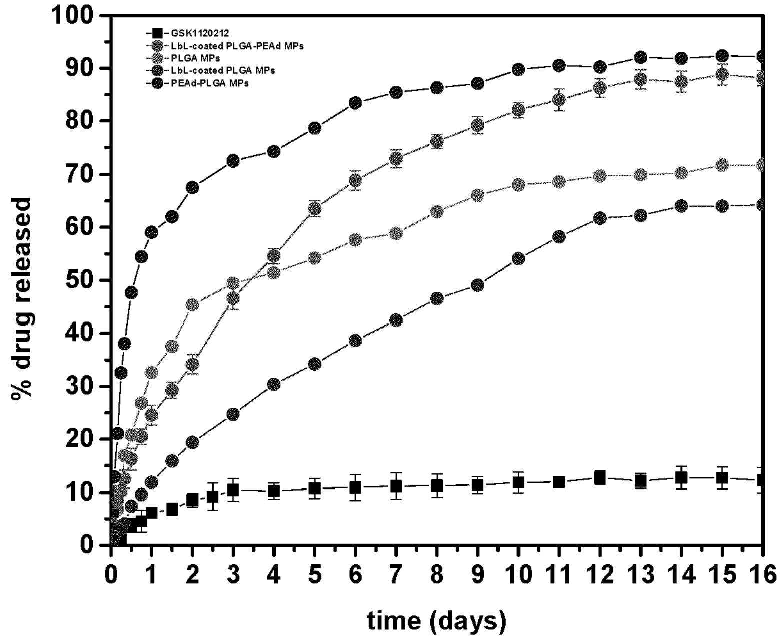


Figure 9

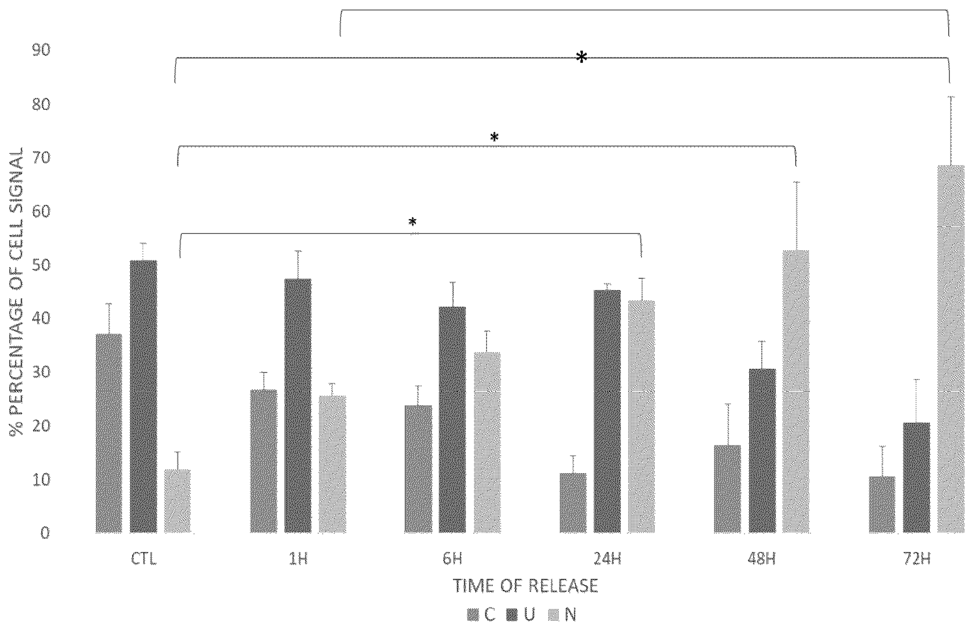


Figure 10

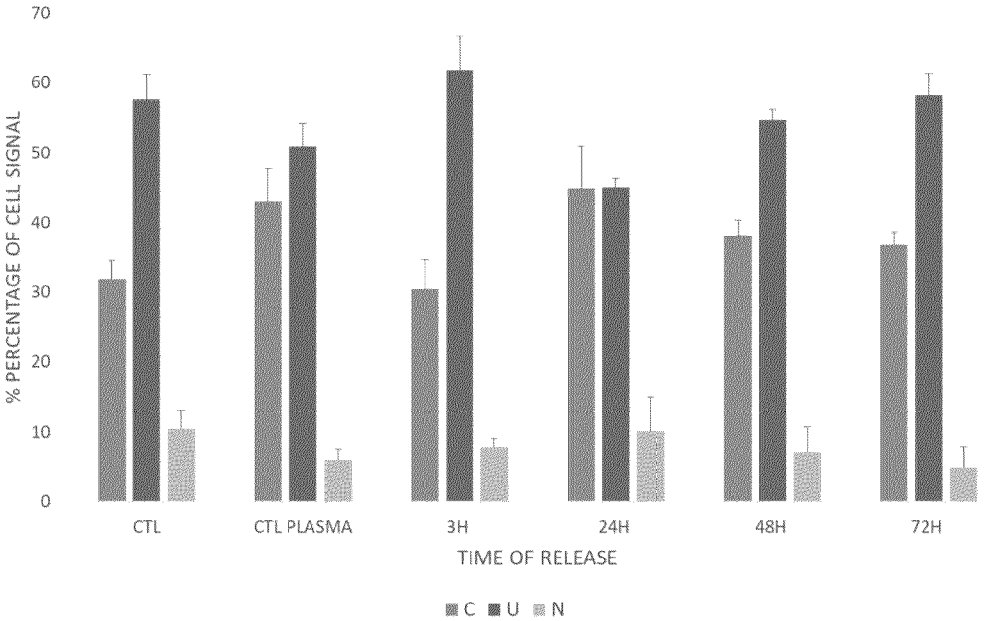
70
60
50
40
30
20
10
0
% PERCENTAGE OF CELL SIGNAL
CTL
CTL PLASMA
3H
24H
48H
72H
TIME OF RELEASE
C
U
N

Figure 11

Europäisches Patentamt
European Patent Office
Office européen des brevets

# EUROPEAN SEARCH REPORT

Application Number
EP 23 02 0447

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 3 791 865 A1 (CAPNOMED GMBH [DE]) 17 March 2021 (2021-03-17) * example 1 * ----- | 1-9 | INV. A61K9/16 A61K9/50 A61K31/519 |
| Y | TSACHOURIDIS KOSTAS ET AL: "Evaluation of poly(lactic acid)/ and poly(lactic-co-glycolic acid)/ poly(ethylene adipate) copolymers for the preparation of paclitaxel loaded drug nanoparticles", JOURNAL OF DRUG DELIVERY SCIENCE AND TECHNOLOGY, vol. 77, 1 November 2022 (2022-11-01), page 103918, XP093129974, FR ISSN: 1773-2247, DOI: 10.1016/j.jddst.2022.103918 * the whole document * ----- | 1-9 | |
| A | KARAVA VASILIKI ET AL: "Poly(l-Lactic Acid)-co-poly(Butylene Adipate) New Block Copolymers for the Preparation of Drug-Loaded Long Acting Injectable Microparticles", PHARMACEUTICS, vol. 13, no. 7, 23 June 2021 (2021-06-23), page 930, XP093130392, CH ISSN: 1999-4923, DOI: 10.3390/pharmaceutics13070930 * the whole document * ----- | 1-9 | TECHNICAL FIELDS SEARCHED (IPC) A61K |

The present search report has been drawn up for all claims

1

| Place of search | Date of completion of the search | Examiner |
|---|---|---|
| Munich | 12 February 2024 | Benbow, Susanne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 23 02 0447

This annex lists the patent family members relating to the patent documents cited in the above-mentioned European search report. The members are as contained in the European Patent Office EDP file on
The European Patent Office is in no way liable for these particulars which are merely given for the purpose of information.

12-02-2024

| Patent document cited in search report | Publication date | Patent family member(s) | Publication date |
|---|---|---|---|
| EP 3791865 A1 | 17-03-2021 | EP 3791865 A1 | 17-03-2021 |
| | | US 2022323368 A1 | 13-10-2022 |
| | | WO 2021052923 A1 | 25-03-2021 |

EPO FORM P0459

For more details about this annex : see Official Journal of the European Patent Office, No. 12/82